# EUROPEAN PATENT APPLICATION

(11) **EP 1 669 056 A2**
(43) Date of publication of application: **14.06.2006**
(21) Application number: 05016529.9
(22) Date of filing: 29.07.2005
(51) Int. Cl.: A61K 8/18

(54) **Oral compositions and systems**

(30) Priority: 12.08.2004 EP 04254845; 12.07.2005 EP 05015068
(71) Applicant: The Procter and Gamble Company, Cincinnati, Ohio 45202 (US)
(72) Inventor: Cahen, Christine Marie, Virginia Water, Surrey GU25 4AN (GB); Moneuze, Gaelle (NMN), Paris 75020 (FR); O'Shea, Edward Patrick John, Chertsey, Surrey KT16 9RB (GB); Potter, Andrea Helen, Old Windsor, Berkshire SL4 2RB (GB); Sarjeant, Danielle Ruth, Twickenham, Middlesex TW2 6SJ (GB); Strand, Ross (NMD), Warfield, Bracknell Berkshire RG42 3RZ (GB)
(74) Representative: Clemo, Nicholas Graham

(57) **Abstract**

The present invention relates to oral compositions, systems and methods of applying oral care compositions to the oral cavity. More specifically, oral compositions having optimized characteristics for application and retention on the oral tissues are provided. Furthermore, systems for applying oral compositions to the oral cavity are provided. A method for treating an oral cavity is further provided that enables chronic application of anti-microbial-containing retentive compositions without generating stain on the oral tissues. Furtherstill, peroxide-stable flavour systems are provided.

## Description

### FIELD OF THE INVENTION

The present invention relates to oral compositions, systems and methods for treating the oral tissues. More specifically, the invention relates to an oral composition and system for sustained contact with the oral tissues and daily use thereof, and a method of application of the oral composition.

### BACKGROUND

The benefits of maintaining oral hygiene are well understood. Consumers understand the benefits of daily oral treatments such as brushing teeth and the use of mouth rinses. These benefits include the reduction of caries, plaque, and gingivitis; treating hypersensitivity; freshening breath; whitening teeth and removing stains; remineralising teeth and the like. An increasing consumer requirement is the need to maintain their teeth for life. Consumers relate healthy oral tissues and "fresh breath" with a healthy body and lifestyle. A wide variety of oral care products have been developed to aid in the short-term maintenance of good oral hygiene. These products deliver various oral care benefit agents to the soft and hard tissues of the oral cavity in such a way that, in general, they are intended for application by the consumer themselves during part of their daily routine, and/or are administered by oral hygiene specialists in the course of administering treatment.

The most frequently used oral care treatments used in the western world are those treatments that are administered by the consumer themselves once or twice a day as part of the daily routine. Examples of such treatments include dentifrices containing for example anti-bacterial plaque actives and/or anti-caries actives and mouth rinses containing anti-bacterial actives and/or breath freshening actives. The existence of "morning breath" and the conditions associated with it indicate that even the application of existing daily oral care regimens prior to retiring in the evening have little effect on the degeneration of oral health overnight. During slumber, reduced salivary flow and excessive growth of anaerobic bacteria result in conditions well suited to the degeneration of the oral tissues and the development of oral malodour and gingivitis. Coupled with reduced pH control in the oral cavity, these conditions are optimal for the development of oral conditions such as caries, gingivitis and plaque formation. Such processes are ongoing, and though they may be reduced or modified by existing treatments, they can only be effectively treated, either prophylactically or therapeutically, by continuous attention, which is impractical, or by the use of long lasting treatments.

Several attempts have been made to provide products having enhanced substantivity and prolonged oral tissue contact times with the objective of increasing the exposure of oral care benefit agents to the oral tissues. These attempts include the use of water-soluble and -insoluble film forming polymers to deliver various actives to the oral tissues, specifically the hard tissues. US 5,462,728 teaches a water insoluble bioadhesive co-polymer matrix containing a therapeutic agent to be applied to the oral cavity. Such water insoluble vehicles are designed to precipitate the polymeric carrier and active agent contained therein upon application to the oral cavity, and are designed for treatment of small areas of the oral cavity. Also, such polymeric films require removal by mechanical means such as brushing. This may also result in a palpable hard coating being formed on the oral tissues that is unpleasant to the consumer. US 5,462,728 further discloses a method of applying the oral composition to the oral cavity resulting in the in situ formation of an adhering, water insoluble film that remains active for a period of hours.

US 5,425,953 teaches the application of compositions comprising cellulosic polymers with high levels of ethyl alcohol and carbamide peroxide. Following application, the solvent evaporates, leaving a polymeric film on the teeth delivering the contained carbamide peroxide. The compositions therein are disclosed as being for intermittent or acute application in the treatment of oral conditions. These compositions contain levels of monohydric alcohols above 50% and may cause undesired consumer reactions to palpable layers on the oral tissues. Furthermore, they may require mechanical means of removal following application.

US 5,438,076 teaches the use of acrylic polymers to deliver pharmacological agents to the oral cavity. These compositions are designed for application to afflicted areas of the oral cavity, not the oral cavity as a whole, and may result in palpable film formation that some consumers find unpleasant.

Another method of prolonged delivery of oral care benefit agents is described in WO 02/34221. This document discloses the application of oral care compositions comprising a silicone resin, a silicone gum and a silicone fluid and an oral care benefit agent. The oral care compositions disclosed by this document form a substantive film on the surface of the teeth or gums and may be "broadly applied to the whole cavity". Due to the composition's substantivity it will remain on the oral tissues for up to 8 hours and requires removal by mechanical means such as brushing or rinsing. Whilst the compositions of WO 02/34221 are excellent for providing long-term delivery of oral care benefits, it has been found that some consumers prefer not to have palpable silicone residues on the tissues of the oral cavity the following morning.

US 5,631,000 teaches the whitening of teeth with an aqueous gel that is exposed to the oral tissues by being placed in a dental tray that is then worn in the oral cavity. The dental tray is usually worn at night, but may be worn during the day. However, dental trays are uncomfortable to wear. Application of oral care overnight without the requirement of a dental tray is advantageous due to the ease of application to the oral cavity, and the good aesthetic experience of the consumer.

WO 04/017933 discloses compositions and methods of use for overnight application and delivery of oral care benefit agents. Whilst providing excellent substantivity, anti-microbial activity and improved oral health, these compositions are not ideally suited for chronic use. Unfortunately, such compositions, due to their long retention times in the oral cavity, and in particular in contact with oral hard tissues, may generate noticeable staining following chronic use; i.e. daily application for at least a week.

It is desirable to have oral compositions that are suitable for over night application that delivers an oral care benefit agent to a consumer whilst sleeping without the requirement of further application or intervention following the initial application and without any staining of oral tissues. Overnight delivery of oral care would be a suitable remedy to combat the conditions in the oral cavity that develop whilst asleep. Overnight delivery is also advantageous as it is easily incorporated into the every day oral regimen of the consumer without excessive requirement for specialist equipment or knowledge.

Furthermore, it is desirable that the oral care product has a pleasant mouth feel and taste acceptable for long term use in the oral cavity. Acceptable mouth feel is advantageous as it encourages regular consumer usage. Unfortunately, many flavour ingredients used in oral compositions are not stable in the presence of other oral care actives, resulting in the flavour components being destabilised and lost from the composition over time. It is desirable to provide oral compositions that have stable flavour systems that produce a consumer-noticeable taste in the presence of peroxide sources.

Long-term mouth feel is recognised as a balancing act between substantivity, adherence and viscosity. Desirable products require sufficient substantivity and viscosity to enable application to the oral cavity, to adhere to the oral tissues and to release the contained oral care benefit agents over an extended period of time. However, the viscosity should not be so high that the consumer can feel globular portions of the newly applied product that have not spread well over the oral tissues upon application. It is desirable to have a gel for use in the present invention that enables easy application to the oral cavity, thin layer formation over the oral tissues and even spread into periodontal pockets and fissures.

It is a consumer need to awake with a "fresh" mouth feel in the morning, but without oral care products palpably maintained on the oral tissues. Therefore, there is a need for oral care products that satisfactorily deliver oral care benefit agents to the oral tissues overnight, but dissolve within the oral cavity such that, once the consumer awakes, he or she does not feel the presence of the applied product, and the product does not require mechanical means of removal such as brushing or rinsing.

### SUMMARY

In a first aspect, the present invention provides an oral care composition in the form of an aqueous gel comprising:
a) an anti-microbial agent;
b) a thickener comprising polysaccharide thickeners, synthetic copolymers or mixtures thereof;
c) from 0.1 % to 1.5% hydrogen peroxide;
d) less than 10% silicone; and
e) less than 18% monohydric alcohols.

In a second, separate aspect, the present invention further provides an oral care composition comprising a peroxide source and a flavour compound, the flavour compound comprising, by weight of the total composition;
a) from 0.1% to 1% of a sweetener comprising saccharin, sucralose or mixtures thereof; and
b) from 0.05% to 1% of a coolant comprising WS-3, WS-23 or mixtures thereof.

In a third, separate aspect, the present invention further provides an oral care system comprising;
a) an oral care composition in the form of an aqueous gel;
b) an applicator.

The applicator can be, for example, a soft-bristled brush or a tube with an elongate, preferably flexible, nozzle, which can be used to deliver the product directly to the gum line, for example, by squeezing the tube. In a preferred embodiment the applicator comprises an elongate handle member having a first free end portion and second end portion; and an applicator head member formed together with the second end portion of said elongate handle member, the head member including a resilient massaging element comprising an elastomeric material having a Shore A hardness of from 20 to 90.

In a fourth, separate aspect, the present invention further provides a method of treating the oral cavity comprising applying an aqueous gel comprising a thickener, from 0.01 % to 5% of at least one anti-microbial agent and from 0.01% to 8% of at least one peroxide-source, wherein the gel is applied daily for at least one week, the gel being such that it remains in contact with the oral tissues for at least 15 minutes.

These and other features, aspects, and advantages of the present invention will become evident to those skilled in the art from a reading of the present disclosure.

### DETAILED DESCRIPTION

While the specification concludes with claims that particularly point out and distinctly claim the invention, it is believed the present invention will be better understood from the following description.

All percentages are by weight of total composition unless specifically stated otherwise and all measurements are made at 20°C, unless otherwise stated. All ratios are weight ratios unless specifically stated otherwise.

Viscosity of the gel as used herein unless otherwise stated is measured using a rheometer with a gap of 500µm, stainless steel parallel plates and continuous linear ramps of shear rates from 0.1 to 1 s⁻¹ and 1 to 900 s⁻¹ run over 60 s using 0.1 cm³ of product at 20°C.

Herein, "thickener" means any material that when added to a solvent or carrier results in the viscosity of the solvent or carrier increasing.

Herein, "abrasive" means any particulate material with polishing or abrasive characteristics that is substantially insoluble in water and has a diameter of from about 1 µm to about 100 µm.

The term "oral care benefit agent" as used herein refers to any composition which has a prophylactic, therapeutic or cosmetic benefit either directly within the oral cavity or which is absorbed via the oral cavity but which has its primary benefits elsewhere.

The term "oral cavity" as used herein refers to the cavity from the lips to the epiglottis. The "hard tissues" comprise tissues such as the teeth and periodontal support and the like, and the "soft tissues" comprise tissues such as the gums, the tongue, the surfaces of the buccal cavity and the like. Within the scope of this application the hard tissues of the oral cavity should also be considered to comprise any devices which are used therein for example dentures, partial dentures, braces and the like.

Active and other ingredients useful herein may be categorised or described herein by their cosmetic and/or therapeutic benefit or their postulated mode of action. However, it is to be understood that the active and other ingredients useful herein can, in some instances, provide more than one cosmetic and/or therapeutic benefit or operate via more than one mode of action. Therefore, classifications herein are made for the sake of convenience and are not intended to limit an ingredient to the particularly stated application or applications listed.

Herein, "comprising" means that other steps and other ingredients which do not affect the end result can be added. This term encompasses the terms "consisting of" and "consisting essentially of'. The compositions and methods/processes of the present invention can comprise, consist of, and consist essentially of the essential elements and limitations of the invention described herein, as well as any of the additional or optional ingredients, components, steps, or limitations described herein.

### A. ORAL COMPOSITION

In a first aspect of the present invention, an oral composition is provided comprising
a) an anti-microbial agent
b) a thickener comprising polysaccharide thickeners, synthetic copolymers or mixtures thereof;
c) from 0.1% to 1.5% hydrogen peroxide;
d) less than 10% silicone; and
e) less than 18% monohydric alcohols.

The oral composition according to the first aspect of the present invention may be applied to the oral cavity by a consumer daily for an extended period of time (i.e. chronic application) without adversely affecting the oral tissues. Furthermore, the oral compositions herein adhere to the oral tissues for an extended period of time, and are well suited for application prior to retiring for sleep, the composition remaining in contact with the oral tissues whilst sleeping. This enables the delivery of superior anti-microbial activity, without any staining associated with long term exposure to anti-microbial agents. Furthermore, the oral compositions herein, when applied before sleeping, allow the consumer to wake with a clean-feeling mouth and avoid the "morning mouth" described above.

### Antimicrobial Agents

The compositions according to the first aspect of the present invention comprise antimicrobial agents known to those skilled in the art, including cationic agents, non-cationic agents and metal ion salts. Such agents may include, but are not limited to, 5-chloro-2-(2,4-dichlorophenoxy)-phenol, commonly referred to as triclosan, and described in The Merck Index, 11th ed. (1989), pp. 1529 (entry no. 9573); phthalic acid and its salts, substituted monoperphthalic acid and its salts and esters, preferably magnesium monoperoxy phthalate, chlorhexidine (Merck Index, no. 2090), alexidine (Merck Index, no. 222; hexetidine (Merck Index, no. 4624); sanguinarine (Merck Index, no. 8320); benzalkonium chloride (Merck Index, no. 1066); salicylanilide (Merck Index, no. 8299); domiphen bromide (Merck Index, no. 3411); cetylpyridinium chloride (CPC) (Merck Index, no. 2024; tetradecylpyridinium chloride (TPC); N-tetradecyl-4-ethylpyridinium chloride (TDEPC); octenidine; delmopinol, octapinol, and other piperidino derivatives; nicin preparations; zinc/stannous ion agents; antibiotics such as augmentin, amoxicillin, tetracycline, doxycycline, minocycline, and metronidazole; and analogs and salts of the above; essential oils including thymol, geraniol, carvacrol, citral, hinokitiol, eucalyptol, catechol (particularly 4-allyl catechol) and mixtures thereof; methyl salicylate; hydrogen peroxide; nanochitosan, metal salts of chlorite and mixtures of all of the above. Preferred antimicrobial agents comprise cetyl pyridinium chloride, triclosan, or mixtures thereof, more preferably cetylpyridinium chloride. Preferably the anti-microbial agent comprises from about 0.01% to about 5%, more preferably from about 0.1% to about 2%, more preferably from greater than about 0.1 % to less than about 1% by weight of the composition.

### Thickeners

The oral compositions herein can be in the form of a gel. The gel is a high viscosity matrix formed from thickeners known in the art which are safe for oral use and do not react with or inactivate the oral care benefit agents incorporated into them. Furthermore, the gel formed with these thickeners may provide sufficient adhesive attachment to the teeth or mucosa to keep them coated for a period of not less than 15 minutes.

The amount of thickener required to form the gel is such that the viscosity of the gel is greater than about 10 Pa.s at a shear rate of 0.1 s⁻¹. This development produces a gel that, when placed on an applicator or finger, does not run off or prove too runny to use effectively. The amount of thickener is such that the viscosity is from about 0.1 Pa.s to about 300 Pa.s, preferably from about 30 Pa.s to about 200 Pa.s, and more preferably from about 80 Pa.s to about 120 Pa.s at a shear rate of 1 s⁻¹. This is advantageous to create a gel with good aesthetics and consumer compliance, and enable the gel to be spread effectively across the oral tissues, yet remain substantive on those tissues following application.

Suitable thickening agents useful in the present invention include polysaccharide thickeners, clays, cross-linked poly-acrylates, polymers, co-polymers, polyethylene glycols and derivatives, protein thickeners and mixtures thereof. Preferred levels of thickener to form the gel are from about 0.1 % to about 5%, preferably from about 2% to about 5%, by weight.

Polysaccharide thickeners useful in the present invention include hydroxylpropyl-methylcellulose (HPMC), hydroxypropylcellulose (HPC), hydroxyethylcellulose (HEC), carboxymethylcellulose (CMC, cellulose gum), methylcellulose, cetylhydroxyethylcellulose, methylhydroxyethylcellulose, microcrystalline cellulose, hydroxyethylethylcellulose, methylhydroxypropylcellulose, carboxymethylhydroxyethylcellulose, xanthan gum, sclerotium gum, carboxymethyl hydroxypropyl guar, guar gum, glyceryl alginate, guar (cyanopsis tetragonoloba) gum, guar hydroxypropyltrimonium chloride, gum arabic/gum acacia, hydroxypropyl guar, karaya (sterculia urens) gum, gellan gum, agar, carrageenan (kappa, iota, lambda), pectin, locust bean (ceratonia siliqua) gum, carboxymethyl chitosan, hydroxyethyl chitosan, carboxymethyl dextran, corn (Zea mays) starch, dextrin, potassium alginate, potato starch modified, propylene glycol alginate, sodium carboxymethyl betaglucan, sodium carboxymethyl dextran, sodium carboxymethyl starch, sodium hydroxypropyl starch phosphate, maltodextrin, algin/alginic acid, and mixtures thereof.

Clays useful in the present invention include sodium magnesium silicate, lithium magnesium silicate, lithium magnesium sodium silicate, sodium magnesium fluorolithosilicate, bentonite, montmorillonite clay and mixtures thereof.

Cross-linked polyacrylates useful in the present invention include sodium acrylate/vinyl alcohol copolymer, acrylate/c10-30 alkyl acrylate crosspolymer, acrylates/ceteth-20 itaconate copolymer, acrylates/ceteth-20 methacrylate copolymer, acrylates/steareth-50 acrylate copolymer, acrylates/steareth-20 itaconate copolymer, acrylates/steareth-20 methacrylate copolymer, carbomer, glycerin/glyceryl polyacrylate and mixtures thereof. Other synthetic polymers and copolymers useful in the present invention include Poloxamer 407, PVM/MA co-polymer, (commercially available under the trade name "Gantrez"), PVP (poly(vinylpyrrolidone)), polyacrylamideomethylpropane sulfonic acid and mixtures thereof.

Polyethylene glycols useful in the present invention include PEG-2M, PEG-5M, PEG-7M, PEG-9M, PEG-14M, PEG-20M, PEG-23M, PEG-25M, PEG-45M, PEG-90M, PEG-115M, PEG-160M, PEG-crosspolymer, PEG-140 glyceryl tristearate and mixtures thereof.

Preferred thickeners for use in the present invention are the polysaccharide thickeners and the synthetic polymers and co-polymers. More preferred are the water-soluble cellulosic and acrylic thickeners. Most preferred is HEC. The aqueous gel may comprise from about 2.1% to 4.9% HEC, preferably from 2.5% to 4.7% and more preferably from 2.8% to 4.3% by weight. It has been found that some oral care benefit agents react with thickeners to modify the viscosity of the gel synergistically. More specifically, it has been found that combinations of quaternary anti-microbials with cellulosic thickeners thicken the gel more effectively than when cellulosic thickeners are used on their own. Preferred are the cellulosic derivatives such as e.g. HPMC and HEC and cationic surfactant antimicrobials. Preferred is the combination of greater than 0.02% cetylpyridinium chloride (CPC) and from about 2.1 % to about 4.9% HEC. Incorporation of CPC with HEC at these levels results in a marked increase in the viscosity of the gel when compared with HEC alone. Without wishing to be bound by theory, it is thought that when the levels of CPC reach the critical micelle concentration, which in the literature is reported to be about 0.017% by weight, the CPC interacts with thickening agents such as HPMC and HEC to thicken the gel significantly more than HPMC or HEC alone.

### Peroxide Source

The oral compositions herein preferably comprise a peroxide source. In addition to providing anti-microbial benefits itself, the peroxide source helps avoid staining of the teeth by the anti-microbial. The peroxide source can be any form that liberates peroxide either by solubilization or hydration. All peroxide active concentrations expressed herein are for hydrogen peroxide, and appropriate conversions must be made for other peroxide liberating molecules such as carbamide peroxide etc. Preferably, the oral compositions herein comprise from about 0.01% to about 8% peroxide source, more preferably from about 0.1 % to about 5%, more preferably still from about 0.1 % to about 2% by weight of the total composition. Suitable examples of peroxide-sources for use herein include hydrogen peroxide, calcium peroxide, carbamide peroxide, sodium percarbonate, benzoyl peroxide or mixtures thereof. Preferably, the peroxide source comprises hydrogen peroxide, calcium peroxide, carbamide peroxide, or mixtures thereof, more preferably hydrogen peroxide. More preferably still, the peroxide source is hydrogen peroxide. Peroxide concentrations can be measured using the iodometric titration method ("Hydrogen Peroxide", Walter C. Schumb, Reinhold Publishing, copyright 1955). The iodometric titration method is a standard method known in the art for measuring peroxide concentration. In general, the method is performed by weighing the strip of material and composition containing the peroxide active, dissolving the composition in 1M sulfuric acid, and reacting the peroxide with an excess of 10% potassium iodide aquesous solution (granular reagent available from J. T. Baker cat no. 3162-01, CAS no. 7681-11-0) in the presence of a few drops of 1% ammonium molybdate (VWR cat no. VW3627-1,). This is then titrated with a 0.025N concentration of sodium thiosulfate (VWR cat. No. VW3127- 1) to a clear endpoint using a starch indicator. The 1% starch indicator (VWR cat no. VW3368-1) is added when the titration solution is a pale yellow. The strip of material is weighed upon completion of the titration and the composition weight is determined by difference from the starting weight of the device plus the weight of the composition. The peroxide concentration in the composition can then be calculated.

If the peroxide concentration is measured after a period of storage of the tooth whitening product and the storage period is long, the concentration of the peroxide active can alternatively be determined by measuring the concentration as described above after at least one hundred and twenty days and then extrapolating for the remainder of the period using first order kinetics, as is known in the art. The above-described method can be performed just after manufacture of a peroxide product and at the end of the specified storage period in order to determine the absolute peroxide concentrations as well as the percentage of the original concentration remaining, as is known in the art.

### B. PEROXIDE STABLE FLAVOURS

In the second aspect of the present invention, oral compositions comprising a peroxide stable flavour system are provided. The flavours systems herein are stable for at least one month at 40oC in the presence of a peroxide-source. Furthermore, the flavour systems herein are consumer acceptable, and provide improved cooling and sensate experience when in the mouth. In addition, the flavour systems herein are able to mask the bitter flavour of cationic anti-microbials such as cetylpyridinium chloride.

According to the second aspect of the present invention, oral care compositions comprise a peroxide source and a flavour compound, the flavour compound comprising, by weight of the total composition;
a) from about 0.1 % to about 1% of a sweetener comprising saccharin, sucralose or mixtures thereof; and
b) from about 0.05% to about 1% of a coolant comprising N-ethyl-p-menthan-3-carboxamide (commercially available as WS-3), N,2,3-trimethyl-2-isopropylbutanamide (commercially available as WS-23), or mixtures thereof.

Preferably, the oral composition comprises from about 0.1% to about 0.4% of the sweetener by weight of the total composition; the sweetener preferably comprises a mixture of saccharin and sucralose. As used herein, the term "saccharin" includes the free acid of saccharin, as well as the alkali metal, alkali earth metal and ammonium salts thereof. In this second aspect the oral composition preferably comprises from about 0.1 % to about 0.5% coolant, by weight of the total composition; the coolant preferably comprising a mixture of N-ethyl-p-menthan-3-carboxamide (WS-3) and N,2,3-trimethyl-2-isopropylbutanamide (WS-23).

Additional flavour materials may be used to further improve the flavour system of the second embodiment. Suitable flavour materials are preferably stable in the presence of a peroxide-source. Non-limiting examples of suitable flavour materials include 1menthol, menthone, menthyl acetate, dihydroanethole, or mixtures thereof. Preferably, the oral composition comprises from about 0.01% to about 5% of these additional flavour materials, more preferably from about 0.01% to about 1%. Additional coolants may comprise 3-1-menthoxypropane-1,2-diol, known as TK-10 (commercially available from Takasago), menthone glycerol acetal, known as MGA (commercially available from Haarmann and Reimer), menthyl lactate, known as Frescolat® (commercially available from Haarmann and Reimer) or mixtures thereof.

Preferably, the oral compositions according to the second aspect may further comprise an anti-microbial agent as disclosed above. Additionally, the oral compositions according to the second aspect may preferably comprise a thickener as described herein.

### C. ORAL CARE SYSTEM

According to a third aspect of the present invention, an oral care system is provided that comprises an oral care composition in the form of an aqueous gel, and an applicator. The applicator can enable better targeting of the oral care composition to the gum line and more even spreading of the composition than application by a user's finger. It is also more hygienic than using a finger.

### Oral Care Composition

The oral care system according to the third aspect of the present invention comprises an oral composition, the composition comprising at least one oral care benefit agent. Preferred oral care compositions are those according to the first and second aspects of the invention. Oral care benefit agents of the present invention may be selected from, in addition to the anti-microbial agents disclosed above, desensitising agents, anti-stain agents, anti-tartar agents, anti-plaque agents, fluoride ion sources, tooth strengthening agents, nutrients, antioxidants, H-2 antagonists and mixtures thereof. The oral care benefit agent may comprise from about 0.01% to about 15% by weight of the gel. The following is a non exclusive list of oral care benefit agents that may be used in the present invention:

### Anti-tartar agents

Anti-tartar agents known for use in dental care products include pyrophosphates, linear polyphosphates with 4 or more repeat units, polyphosphonates and mixtures thereof. Pyrophosphate ions delivered to the teeth are derived from pyrophosphate salts. The pyrophosphate salts are described in more detail in Kirk & Othmer, *Encyclopedia of Chemical Technology,* Third Edition, Volume 17, Wiley-Interscience Publishers (1982). Agents that may be used in place of or in combination with pyrophosphate salts include such known materials as synthetic anionic polymers including polyacrylates and copolymers of maleic anhydride or acid and methyl vinyl ether, as described, for example, in U.S. Patent 4,627,977, to Gaffar et al.; as well as, e.g., polyamino propoane sulfonic acid (AMPS), zinc citrate trihydrate, linear polyphosphates (e.g., tripolyphosphate; hexametaphosphate), diphosphonates (e.g., ethane-1-hydroxy-1,1-diphosphonate, 1-azacycloheptane-1,1-diphosphonate), polypeptides (such as polyaspartic and polyglutamic acids), and mixtures thereof. Further antitartar agents include polycarboxylates; polyepoxysuccinates; ethylenediaminetetraacetic acid; linear alkyl diphosphonates; linear carboxylic acids; sodium zinc citrate, nitrilotriacetic acid and related compounds.

### Fluoride Ion Source

Fluoride ion sources are well known for use in oral care compositions as anticaries agents. Fluoride ions are contained in a number of oral care compositions for this purpose. A wide variety of materials can be employed as sources of soluble fluoride in the instant compositions. Examples include sodium fluoride, stannous fluoride and sodium monofluorophosphate. Suitably the compositions provide from about 50 ppm to 10,000 ppm, more preferably from about 100 to 3000 ppm, of fluoride ions by weight.

### Anti-inflammatory Agents

Anti-inflammatory agents can also be present in the aqueous gel of the present invention. Such agents may include, but are not limited to, non-steroidal anti-inflammatory agents (or NSAIDs) such as ketorolac, flurbiprofen, ibuprofen, naproxen, indomethacin, aspirin, ketoprofen, piroxicam and meclofenamic acid. Use of NSAIDs such as Ketorolac are claimed in U.S. Patent 5,626,838, issued May 6, 1997. Disclosed therein are methods of preventing and, or treating primary and reoccurring squamous cell carcinoma of the oral cavity or oropharynx by topical administration to the oral cavity or oropharynx an effective amount of an NSAID.

### Nutrients

Nutrients may improve the condition of the oral cavity and can be included in the compositions herein. Nutrients include minerals, vitamins, nutritional supplements, and mixtures thereof.

Suitable minerals include calcium, phosphorus, fluoride, zinc, manganese, potassium and mixtures thereof. These minerals are disclosed in Drug Facts and Comparisons (loose leaf drug information service), Wolters Kluer Company, St. Louis, Mo., ©1997, pp10-17.

Vitamins can be included with minerals or used separately. Vitamins include Vitamins C and D, thiamine, riboflavin, calcium pantothenate, niacin, folic acid, nicotinamide, pyridoxine, cyanocobalamin, para-aminobenzoic acid, bioflavonoids, and mixtures thereof. Such vitamins are disclosed in Drug Facts and Comparisons (loose leaf drug information service), Wolters Kluer Company, St. Louis, Mo., ©1997, pp. 3-10.

Nutritional supplements include amino acids, lipotropics, fish oil, protein products, glucose polymers, corn oil, safflower oil, medium chain triglycerides and mixtures thereof, as disclosed in Drug Facts and Comparisons (loose leaf drug information service), Wolters Kluer Company, St. Louis, Mo., ©1997, pp. 54-54e. Amino acids include, but, are not limited to L-tryptophan, L-lysine, methionine, threonine, levocarnitine or L-carnitine and mixtures thereof. Lipotropics include, but are not limited to choline, inositol, betaine, linoleic acid, linolenic acid, and mixtures thereof. Fish oil contains large amounts of omega-3 (N-3) polyunsaturated fatty acids, eicosapentaenoic acid and docosahexaenoic acid.

### Enzymes

An individual or combination of several compatible enzymes can be included in the compositions herein. Enzymes are biological catalysts of chemical reactions in living systems. Enzymes combine with the substrates on which they act forming an intermediate enzyme-substrate complex. This complex is then converted to a reaction product and a liberated enzyme which continues its specific enzymatic function.

Enzymes useful in the present invention include any of the commercially available proteases, glucanohydrolases, endoglycosidases, amylases, mutanases, lipases and mucinases or compatible mixtures thereof. Preferred are the proteases, dextranases, endoglycosidases and mutanases, most preferred being papain, endoglycosidase or a mixture of dextranase and mutanase.

### Antioxidants

Antioxidants are generally recognized as useful in aqueous gels such as those of the present invention. Antioxidants are disclosed in texts such as Cadenas and Packer, The Handbook of Antioxidants, © 1996 by Marcel Dekker, Inc. Antioxidants that may be included in the aqueous gel or substance of the present invention include, but are not limited to, vitamin E, ascorbic acid, uric acid, carotenoids, vitamin A, flavonoids and polyphenols, herbal antioxidants, melatonin, aminoindoles, lipoic acids and mixtures thereof.

### H-2 Antagonists

Histamine-2 (H-2) receptor antagonist compounds (H-2 antagonists) may be used in the aqueous gel of the present invention. H-2 antagonists are compounds that block H-2 receptors, but do not have meaningful activity in blocking histamine-1 (H-1) receptors. H-2 antagonists stimulate the contraction of smooth muscle from various organs, such as the gut and bronchi; this effect can be suppressed by low concentrations of mepyramine - a typical antihistaminic drug. The pharmacological receptors involved in these mepyramine-sensitive histamine responses have been defined as H-1 receptors (Ash, A.S.F. & H.O. Schild, Brit. J. Pharmacol Chemother., Vol. 27 (1966), p. 427). The H-2 antagonists useful in the aqueous gels are those that block the receptors involved in mepyramine-insensitive, non-H-1 (H-2), histamine responses, and do not block the receptors involved in mepyramine-sensitive histamine responses. H-2 antagonists meeting the above criteria include cimetidine, ranitidine, and others disclosed in US 5,294,433 and US 5,364,616.

### Applicator

A preferred applicator for use herein comprises;
i) an elongate handle member having a first free end portion and second end portion; and
ii) an applicator head member formed together with the second end portion of said elongate handle member, and which includes a resilient massaging element comprising a thermoplastic elastomeric material having a Shore A hardness of from 20 to 90; the applicator head member not comprising any form of bristles having a diameter 0.5mm or less, and length of at least 4mm, preferably at least 5mm.

The applicator head is configured to be comfortably positioned and moved along the inner and outer gum surfaces inside the mouth so as to apply the oral care composition to the oral hard and soft tissues. Suitable, non-limiting examples of applicators are described in more detail in WO 03/086141.

The applicator includes an elongate handle member having a first free end portion and a second end portion. The applicator also includes an applicator head member formed together with the second end portion of the elongate handle member. Preferably, the applicator head element includes a resilient massaging element formed so as to generally adapt to the contours of a gum portion having the oral composition applied thereto, when pushed thereagainst. The resilient massaging element comprises a thermoplastic elastomeric material having a shore A hardness of from 20 to 90, preferably from 20 to 80, more preferably from 20 to 75. As used herein, Shore A hardness is measured according to ASTM D2240-00, revised 10 January 2002.

The elongate handle member may be formed so as to facilitate attachment of the applicator to an electrically driven oscillatory device such that the oscillatory device provides an oscillatory movement to the applicator head member.

The applicator head member may generally be configured as a bell shape, having a recessed inner surface terminating in an outer lip. The outer lip element is formed having a curved outer edge. This is beneficial to enable the oral care composition to be dispensed and retained thereon.
Figure 1 illustrates a head-on view of an applicator in accordance with a preferred embodiment of the present invention;
Figure 2 illustrates a side view of the applicator of Figure 1
Figure 3 illustrates a plan view of the applicator of Figure 1 ;
Figure 4 is a plan view of an alternative, elastomeric head member for the applicator;
Figure 5 is a sectional view of the head region of an applicator;

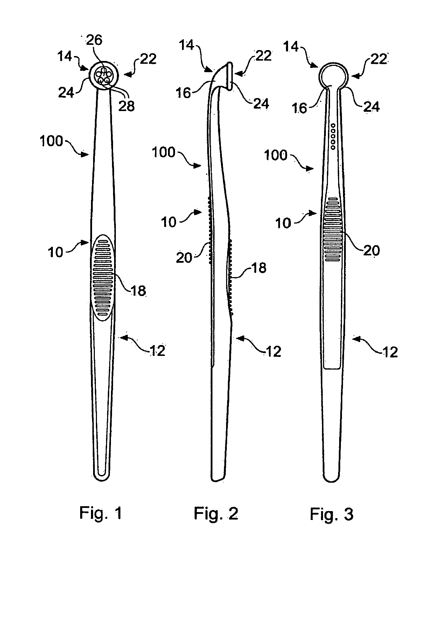

Figures 1-3 show various views of an applicator generally referenced 100, in accordance with a preferred embodiment of the present invention. Applicator 100 includes an elongate handle generally referenced 10 having a free first end portion generally referenced 12 and a second end portion generally referenced 14 including a curved neck portion referenced 16.

There are two ergonomically formed handhold elements referenced 18 and 20 formed integrally with first end portion 12 of elongate handle member 10.

Applicator 100 also includes an applicator head member generally referenced 22, formed together with second end portion 14 of elongate handle member 10 and bonded thereto. Applicator head member 22 is formed of a resilient rubber or plastic material and formed generally having a bell shape having a recessed inner surface terminating in an outer lip referenced 24. Resilient protrusions referenced 28 are formed extending from recessed inner surface 26 so as to improve the massaging contact and application of the oral care composition to the gums and teeth.

The ergonomics of the applicator 100 have been optimized in such a way that the distance from the end of ridge of the user's thumb to the active tip or second end portion 14 of applicator 100 are the average length of one side of the structure of the human gum, therefore allowing the user to apply the oral composition to the rearmost teeth and gums in the mouth.

If seen from the side, as seen in Figure 2, curved neck portion 16 of applicator 100 is curved in order to follow the natural anatomic contour of the human denture. Therefore, when applying the oral composition to the most remote teeth and gum areas, elongate handle member 10 remains generally parallel to the denture, and curved neck portion 16 maintains some distance between elongate handle member 10 and the dentures without affecting functionality.

Curved neck portion 16 is formed with a progressive flexibility needed in order to prevent excessive pressure being applied to the oral tissues while in use. This progressive flexibility of curved neck portion 16 is regulated through the cross-sectional geometry of curved neck portion 16, which becomes progressively thinner towards its extremity 30. The cross-sectional shape of curved neck portion 16 is generally elliptical not only for safety, having no sharp edges, but also to have an optimum compromise between the necessary strength needed in curved neck portion 16 and the need to minimize the net width of curved neck portion 16 while in use.

Handhold element 18 for the user's thumb is positioned generally facing the same direction as applicator head member 22, disposed on first free end 12 of elongate handle member 10. Handhold element 20 for the user's index and other fingers is positioned generally facing the opposite direction to applicator head member 22, so disposed on first free end 12 of elongate handle member 10, to allow for better control and balance while manipulating applicator 100.

Applicator head member 22 is formed having a generally bell shape, and is attached by means of either a chemical bond or a mechanical bond to extremity 30 of second end 14 of elongate handle member 10. Applicator head member 22 is disposed at an angle of substantially 90 degrees relative to the axis of extremity 30.

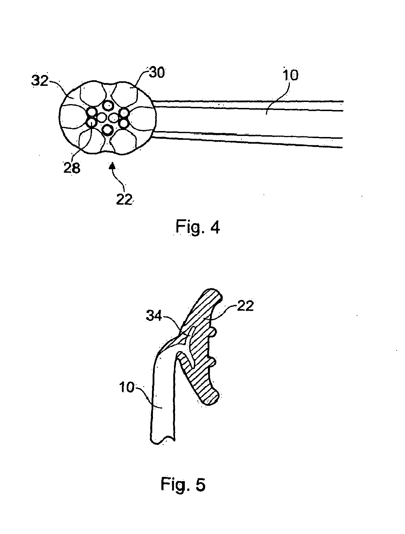

Figure 4 illustrates an alternative head member 22 in which the outer lip broken into a ring of discrete, resilient massaging protrusions 30. Channels 32 are formed between adjacent pairs of massaging protrusions. The channels allow gel applied to the centre of the head member to squeeze out between the massaging protrusions when the applicator is used, preventing the gel being swept around the gums in one block and thus providing more even spreading. Inner protrusions 28 assist in gum massage and help to prevent gel running out from the centre of the head member when the gel is first squeezed onto the applicator.

Figure 5 shows one method of connecting the head member 22 to the handle 10. Handle 10 is moulded with a thin petal-shaped extension 34 which provides increased surface are for attaching the head member securely to the handle without unduly restricting the movement of the head member relative to the handle. Further security of attachment of the elastomeric head member relative to the handle can be provided by providing a hole (not shown) through the handle where the member is attached. When the head member is moulded on elastomer flows through the hole providing a loop of elastomer material which prevents the head member being pulled from the handle in use.

### General

The water present in the oral care compositions according to all aspects of the present invention should preferably be deionized and free of organic impurities. Water typically comprises from about 0.1 % to 95%, preferably from about 5% to about 90%, and most preferably from about 10% to about 80%, by weight of the gel. This amount of water includes the free water that is added plus that introduced with other materials.

The aqueous gel according to all aspects of the present invention may optionally comprise xylitol. Xylitol is a polyol that may be added to provide sweetening and flavouring. Xylitol is believed to have benefits as an anti-caries agent. The aqueous gel may comprise from about 0.1 % to about 15% xylitol, preferably from about 1% to 10%, and more preferably from about 2% to 8% xylitol.

A pH adjusting agent may also be added to optimize the storage stability of the gel and to make the substance safe for oral tissue. These pH adjusting agents, or buffers, can be any material which is suitable to adjust the pH of the aqueous gel. Suitable materials include sodium bicarbonate, sodium phosphate, sodium hydroxide, ammonium hydroxide, sodium stannate, triethanolamine, citric acid, sorbic acid, malic acid, hydrochloric acid, sodium citrate, potassium sorbate, malic acid disodium salt and combinations thereof. The pH adjusting agents are generally added in sufficient amounts to adjust the pH of the gel to from about 3.5 to about 11, preferably from about 4 to about 9, and more preferably from about 4.5 to about 8. pH adjusting agents are generally present in an amount of from about 0.001% to about 15% and preferably from about 0.005% to about 5%.

Humectants can also be added to the aqueous gel according to all aspects of the present invention. Suitable humectants include glycerin, sorbitol, polyethylene glycol, propylene glycol, and other edible polyhydric alcohols. Humectants are generally present in an amount of from about 10% to about 50% and preferably from about 15% to about 40%, by weight of the aqueous gel. In addition to the above materials the gel of the present invention may comprise a number of other components.

In addition, the aqueous gel according to all aspects of the present invention preferably comprises not more than about 18% C₁-C₆ monohydric alcohols. Higher alcohol levels in a gel intended for overnight use are potentially deleterious. However, it is known to those skilled in the art that polyhydric alcohols disclosed above are useful as humectants in gels. Preferably, the aqueous gel contains less than 10% monohydric alcohols, more preferably less than 5%, and more preferably still contains no monohydric alcohols. These levels are desirable to maintain safety and gel aesthetics.

Similarly, the aqueous gel according to all aspects of the present invention preferably comprises less than 5% abrasives. Abrasives, whilst useful in dentifrices, are not desirable in the current invention. Preferably the gel comprises less than 4% abrasives, more preferably less than 2% abrasives and more preferably still comprises no abrasives. The applicant has found that low levels of abrasives are desirable to maintain consumer compliance and good gel aesthetics.

The aqueous gel according to all aspects of the present invention may comprise moderate levels of silicones. Silicones may be desirable to aid the modification of the rheology and substantivity. However, high levels of silicones are undesirable as some consumers would prefer the gel not to be as substantive as gels containing higher levels of silicones. Gels with moderate levels of silicones are desirable as they provide improved mouth feel and sensate delivery. Aqueous gels for use herein preferably comprise less than 10%, preferably from about 0.05% to about 9%, more preferably from about 0.1 % to about 8%, by weight, of a silicone. Suitable silicones for use in the present invention include those disclosed in WO 01/01940. Preferred silicones include silicone resins, silicone gums and silicone fluids having a viscosity, at 25°C, of from about 1x10⁻⁶ m²/s to about 1x10⁻³ m²/s. More preferred are the silicone fluids. More preferred still are the polysiloxane fluids include linear polysiloxane polymers such as the linear dimethicones having a molecular weight of at least 4000 and where R is a methyl substituent, and other low viscosity analogues of the polysiloxane materials. Also preferred are the alkyl and alkoxy substituted dimethicone polyols as disclosed in WO 96/33693.

### D Method of Use

In a fourth aspect, the present invention is directed to a method of treating the oral cavity comprising applying an aqueous gel comprising a thickener, from 0.01% to 5% of at least one anti-microbial agent and from 0.01% to 8% of at least one peroxide source, wherein the gel is applied daily for at least one week, the gel being such that it remains in contact with the oral tissues for at least 15 minutes. Preferably, the application is such that the gel is applied to the oral cavity before sleeping and is not intentionally removed from the oral cavity by way of rinsing or mechanical brushing or other such like means before sleeping. It has been found that this method is advantageous in combating the degeneration of the oral cavity overnight and reducing morning mouth malodour.

The method comprises the application of the aqueous gel to the oral cavity by the consumer as part of the daily oral hygiene regimen after completing brushing, mouth washing, treatment with dental floss and other such like activities associated with the maintenance of oral hygiene. More preferable is the method wherein the application of the aqueous gel to the oral cavity follows the completion of oral hygiene activities by the consumer, and prior to sleeping. Preferably the gel is maintained on, and releases contained oral care benefit agents onto, the tissues of the oral cavity for an extended period of time not less than 15 minutes, preferably not less than 30 minutes and more preferably not less than 1 hour. Preferably the gel is applied daily for at least two weeks, more preferably daily for at least a month, and more preferably still for at least two months.

Preferably, the aqueous gel is applied to the oral cavity according to the method of the fourth aspect of the present invention without the aid of a solid support structure. As used herein "solid support structure" means devices and such like that are used to maintain the aqueous gel in contact with the teeth. For example, dental trays and film-like structures disclosed in WO 98/55044 are solid support structures herein. Preferably the gel is applied to the oral cavity using an applicator as disclosed herein, but is substantive and adheres to the oral tissues by itself.

Preferably, the method of use according to the present invention does not result in significant staining of the oral hard tissues. Without wishing to be bound by theory, it is believed that chronic (i.e. daily) use of compositions for periods of at least one week comprising anti-microbial agents may result in staining of the oral hard tissues. This staining manifests as a yellowish/brown tint on the surface of the teeth that is often quite noticeable. The compositions for use according to the fourth aspect of the present invention include a peroxide source that prevents the stain from becoming noticeable. As used herein "significant staining" means either about 10% consumer noticeability by first person assessment (i.e. at least 10% of subjects self-report staining on their oral hard tissues following daily usage according to the present invention) or statistically significant differences (p<0.05) from a baseline measurement (day 0, immediately prior to starting use according to the invention) using the Lobene Stain Index as published in the Journal of the American Dental Association (1968); 77(4), p. 849-855. The Lobene Stain Index is a well-recognised method for scoring stain intensity and area. Briefly, stain intensity and area are scored on the gingival and body regions of all anterior teeth using a 4-point scale ranging from "0" (no stain) to "3" (intensity "heavy" or area "greater than 2/3 of region") by trained observers. All graded tooth surface values per subject are averaged to produce a single intensity and area score.

The aspects and embodiments of the present invention set forth in this document have many advantages. For example, they can provide increased efficacy of delivery of oral care benefit agents that provide for better oral hygiene. Similarly, overnight treatment of the oral cavity may result in a reduction of "morning mouth" experienced by the consumer. Furthermore, overnight application of oral care benefit agents may result in effective reduction in the degeneration of the oral cavity accelerated by the conditions imparted by sleeping. Various embodiments of the present invention address the need for better consumer aesthetics and appeal of intensive oral treatments combined with increased ease of application.

The following examples, described in Tables A and B, further describe preferred embodiments within the scope of the present invention. The examples are given solely for the purpose of illustration, and are not to be construed as limitations of the present invention since many variations thereof are possible without departing from its scope. The example compositions herein are used daily by applying the gel around the gum line, preferably after cleaning one's teeth, and carrying out one's normal oral care routine prior to retiring for the evening. When used in this manner they provide breath freshness benefits the following morning without noticeable staining of the teeth.

| **Table A** | **Example (% w/w)** | | | |
|---|---|---|---|---|
| Material | **I** | **II** | **III** | **IV** |
| Purified water, USP | q.s. | q.s. | q.s. | q.s. |
| HEC | 2.80¹ | 3.20¹ | 3.20¹ | 3.20¹ |
| Sodium saccharin | 0.20 | 0.20 | 0.20 | 0.20 |
| CPC | 1.00 | 1.00 | 1.00 | 0.50 |
| Propylene glycol | 22.00 | 22.00 | 22.00 | 22.00 |
| Xylitol | 6.00 | 6.00 | 6.00 | 6.00 |
| Hydrogen peroxide | 0.50 | 0.10 | 1.00 | 1.00 |
| Citric acid | - | - | - | 0.020 |
| Sodium citrate | - | - | - | 0.019 |
| Sodium stannate | - | - | - | 0.020 |
| Sucralose | 0.05 | 0.15 | 0.15 | 0.15 |
| WS-3 | 0.20 | 0.20 | 0.20 | 0.20 |
| WS-23 | 0.15 | 0.15 | 0.15 | 0.15 |
| Flavour | 0.80 | 0.80 | 0.80 | 0.80 |
| Viscosity (Pa.s, shear rate 0.1 s⁻¹) | 210.6 | 296.7 | 306.3 | 470.5 |
| Viscosity (Pa.s, shear rate 1.0 s⁻¹) | 140.3 | 199.1 | 195.7 | 214.9 |

| **Table B** | **Example (% w/w)** | | | | |
|---|---|---|---|---|---|
| Material | **V** | **VI** | **VII** | **VIII** | **IX** |
| Purified water, USP | q.s. | q.s. | q.s. | q.s. | q.s. |
| HEC | 3.20² | 3.20³ | 3.20² | 3.201² | 3.20² |
| Sodium saccharin | 0.20 | 0.20 | 0.20 | 0.20 | 0.20 |
| CPC | 0.50 | 0.50 | 0.50 | 0.50 | 0.50 |
| Propylene glycol | 22.00 | 22.00 | 22.00 | 22.00 | 22.00 |
| Xylitol | 6.00 | 6.00 | 6.00 | 6.00 | 6.00 |
| Hydrogen peroxide | 1.00 | 1.00 | 1.00 | 1.00 | 1.00 |
| Citric acid | 0.020 | 0.020 | - | 0.020 | - |
| Sodium citrate | 0.019 | 0.019 | - | 0.019 | - |
| Sodium stannate | 0.020 | 0.020 | 0.020 | 0.020 | 0.020 |
| Sorbic acid | | | 0.046 | - | - |
| Potassium sorbate | | | 0.024 | - | - |
| Malic acid | | | - | - | 0.0275 |
| Malic acid disodium salt | | | - | - | 0.0450 |
| Sucralose | 0.15 | 0.15 | 0.15 | 0.15 | 0.15 |
| WS-3 | 0.20 | 0.20 | 0.20 | 0.20 | 0.20 |
| WS-23 | 0.15 | 0.15 | 0.15 | 0.15 | 0.15 |
| Flavour | 0.80 | 0.80 | 0.80 | 0.80 | 0.80 |
| Viscosity (Pa.s, shear rate 0.1 s⁻¹) | 128.2 | 18.1 | 173.1 | 188.1 | 175.5 |
| Viscosity (Pa.s, shear rate 1.0 s⁻¹) | 74.4 | 14.9 | 74.37 | 80.47 | 87.66 |

| | | | | | |
|---|---|---|---|---|---|
| ¹ HEC Natrosol 250 M-Pharm; | | | | | |
| ² 50%HEC Natrosol 250M-Pharm & 50% HEC Natrosol 250G-Pharm; | | | | | |
| ³HEC Natrosol 250G-Pharm | | | | | |

## Claims

1. An oral care composition in the form of an aqueous gel comprising:
a) an anti-microbial agent;
b) a thickener comprising polysaccharide thickeners, synthetic polymers and copolymers or mixtures thereof;
c) from 0.1 % to 2% hydrogen peroxide;
d) less than 5% abrasive;
e) less than 10% silicone; and
f) less than 18% monohydric alcohols.

2. An oral care composition according to Claim 1 wherein the composition has a viscosity of greater than 10 Pa.s at a shear rate of 0.1s⁻¹.

3. An oral care composition according to Claim 1 or Claim 2 further having a viscosity of from 0.1 Pa.s to 300 Pa.s at a shear rate of 1s⁻¹.

4. An oral care composition according to any one of the preceding claims wherein the anti-microbial agent comprises a cationic anti-microbial.

5. An oral care composition according to Claim 4 wherein the anti-microbial agent comprises cetylpyridinium chloride.

6. An oral care composition according to any of the preceding claims comprising from 0.01% to 5% of the anti-microbial agent.

7. An oral care composition according to any one of the preceding claims comprising from 0.1 % to 2%, preferably greater than 0.1 % to less than 1%, of the anti-microbial agent.

8. An oral care composition according to any one of the preceding claims comprising from 0.1 % to 5% thickener, preferably from 2% to 5%.

9. An oral care composition according to any one of the preceding claims wherein the thickener comprises hydroxyethylcellulose, hydroxypropylmethylcellulose, or mixtures thereof, preferably hydroxyethylcellulose.

10. An oral care composition comprising a peroxide source and a flavour compound, the flavour compound comprising, by weight of the total composition;
a) from 0.1% to 1% of a sweetener comprising saccharin, sucralose or mixtures thereof; and
b) from 0.05% to 1% of a coolant comprising N-ethyl-p-menthan-3-carboxamide, N,2,3-trimethyl-2-isopropylbutanamide or mixtures thereof.

11. An oral care composition according to Claim 10 comprising from 0.01% to 8%, preferably from 0.1% to 5%, more preferably from 0.5% to 2% peroxide source.

12. An oral care composition according to Claim 10 or Claim 11 wherein the peroxide source comprises hydrogen peroxide.

13. An oral care composition according to any one of Claims 10 to 12 comprising from 0.1 % to 0.4% sweetener.

14. An oral care composition according to any one of Claims 10 to 13 comprising from 0.1 % to 0.5% coolant.

15. An oral care composition according to any one of Claims 10 to 14 wherein the coolant comprises a mixture of N-ethyl-p-menthan-3-carboxamide and N,2,3-trimethyl-2-isopropylbutanamide.

16. An oral care composition according to any one of Claims 10 to 15 wherein the sweetener comprises a mixture of sucralose and saccharin.

17. An oral care composition according to any one of Claims 10 to 16 wherein the peroxide source is hydrogen peroxide.

18. An oral care system comprising;
a) an oral care composition in the form of an aqueous gel;
b) an applicator comprising;
i) an elongate handle member having a first free end portion and second end portion; and
ii) an applicator head member formed together with the second end portion of said elongate handle member, and which includes a resilient massaging element comprising an elastomeric material having a Shore A hardness of from 20 to 90; the applicator head member not comprising any form of bristles having a diameter 0.5mm or less, and length of at least 4mm, preferably at least 5mm.

19. An oral care system according to Claim 18 wherein the oral care composition comprises an oral care benefit agent selected from anti-microbial agents, desensitising agents, anti-stain agents, anti-tartar agents, anti-plaque agents, fluoride ion sources, tooth strengthening agents, nutrients, antioxidants, H-2 antagonists or mixtures thereof, preferably anti-microbial agents.

20. An oral care system according to Claim 19 wherein the oral care composition comprises from 0.01% to 5% oral care benefit agent.

21. An oral care system according to Claim 19 or Claim 20 wherein the oral care composition comprises from 0.1% to 1.5% cetylpyridinium chloride.

22. An oral care system according to any one of Claims 18 to 21 wherein the oral care composition further comprises a peroxide source.

23. An oral care system according to Claim 23 wherein the peroxide source comprises from 0.1% to 5%, by weight of the oral care composition, hydrogen peroxide, preferably from 0.1 % to 2% hydrogen peroxide.

24. An oral care system according to any one of Claims 18 to 23 wherein the oral care composition has a viscosity of from 0.1 Pa.s to 300 Pa.s at a shear rate of 1s⁻¹.

25. A method of treating the oral cavity comprising applying an aqueous gel comprising a thickener, from 0.01% to 5% of at least one anti-microbial agent and from 0.01% to 8% of at least one peroxide-source, wherein the gel is applied daily for at least one week, the gel being such that it remains in contact with the oral tissues for at least 15 minutes.

26. The method according to Claim 25 wherein the aqueous gel is applied to the oral cavity without the aid of a solid support structure.

27. The method according to Claim 25 or Claim 26 wherein the gel has a viscosity of from 0.1 Pa.s to 300 Pa.s at a shear rate of 1s⁻¹.

28. The method according to any one of Claims 25 to 27 wherein the gel comprises less than 5% abrasive, preferably less than 1% abrasive.

29. The method according to any one of Claims 25 to 28 wherein the gel comprises less than 18% monohydric alcohols.

30. The method according to any one any one of Claims 25 to 29 wherein the gel comprises less than 10% silicone.

31. The method according to any one of Claims 25 to 30 wherein the gel is applied daily for at least two weeks, preferably at least a month.
